Europäisches Patentamt

European Patent Office

Office européen des brevets

(11) Publication number: 0 392 808 A2

# EUROPEAN PATENT APPLICATION

(21) Application number: 90303863.6

(22) Date of filing: 10.04.90

(51) Int. Cl.5: G01N 33/58, G01N 33/533, G01N 33/573, G01N 33/543, C12Q 1/00

The application is published incomplete as filed (Article 93 (2) EPC). The point in the description or the claim(s) at which the omission obviously occurs has been left blank.

(30) Priority: 10.04.89 US 335689

(43) Date of publication of application: 17.10.90 Bulletin 90/42

(84) Designated Contracting States: AT BE CH DE DK ES FR GB GR IT LI LU NL SE

(71) Applicant: **The University of Georgia Research Foundation, Inc. Boyd Graduate Studies Research Center US-Athens, Georgia 30602(US)**

(72) Inventor: **Cummings, Richard D. Route 4 Box 4013 Danielsville Georgia 30633(US)** Inventor: **Cormier, Milton J. Route 1 Box 34A Bogart Georgia 30622(US)**

(74) Representative: **Armitage, Ian Michael et al MEWBURN ELLIS 2 Cursitor Street London EC4A 1BQ(GB)**

(54) **Enzyme luminescence assay.**

(57) A method for determining the level of activity of an enzyme in a sample, comprising combining in a reaction medium a sample suspected of containing the enzyme and a labeled substrate for the enzyme capable of reacting with the enzyme to provide a product; allowing a reaction to take place between the enzyme and the substrate, whereby the product is formed in the presence of the enzyme; combining with the reaction medium, either before or after the reaction is allowed to take place, a receptor capable of binding specifically with either the labeled product or the labeled substrate, but not both, to form a complex; and determining the presence or amount of detectable label in the complex or uncomplexed in the reaction medium, wherein the detectable label is a bioluminescent protein.

EP 0 392 808 A2

## ENZYME LUMINESCENCE ASSAY

This invention is directed to a method for detection of enzyme activity and is particularly directed to assays that determine levels of enzyme activity in samples of biological origin.

A wide variety of assays are available for clinical determination of concentrations and activities of specific molecules having biological importance, whether indicative of normal or diseased states. Among the molecules being assayed are enzymes, the prototypical biological catalysts. Although enzymes can be assayed by traditional methods that merely detect whether or not the molecule is present, such assays are not as useful as assays which measure the level of enzyme activity. For example, an immunoassay that relies on the presence of a particular epitope on the enzyme for detection of that enzyme will report the presence of a given amount of enzyme as long as the epitope is unaltered. If the enzyme is present but the enzyme activity has been altered, for example by irreversible binding to a modified substrate or by poisoning from a metal ion present at the active center of the enzyme, inaccurate levels of enzyme activity will be reported even though the amount of enzyme present is correctly reported.

Techniques are currently available that measure enzyme activity as opposed to amount. Since enzymes are catalytic molecules that convert substrates to products, such measurements typically will involve measurement of the change in the amount of either substrate or product over time. One method of making such measurements is to use a substrate that is colorless but which forms a colored product when acted upon by the enzyme. A number of such reactions have been developed for use with enzymes that are themselves used as labels in analytical reactions, such as the enzyme-linked immunosorbent assay (ELISA). Peroxidase enzymes are often used as labels of this type, and it is therefore easy to measure the activity of peroxidase enzymes using the same substrates and reaction products. However, such assays are useful only if a suitable substrate that undergoes a color change is available and are therefore not generally available.

For most types of enzymes, color-developing assays are not available. Animal-cell glycosyltransferases are examples of one such class of enzymes. These are enzymes that catalyze the formation of glycosydic linkages by transfer of a sugar moiety from a sugar nucleotide doner to a specific hydroxyl group of a growing polysaccharide or to a growing oligosaccharide chain on a glycoconjugate. Representative glycoconjugates in animal cells include glyoproteins, glycolipids, and glycosaminoglycans.

Unlike nucleic acids and proteins, there is no template involved in the synthesis of glycoconjugates; therefore, the control of their synthesis and the fidelity of their structure is dependent upon the expression and exquisite specificity of glycosyltransferases. The total number of different glycosyltransferases in animal cells is not known; however, based on the variety of glycoconjugate structures observed, there appear to be hundreds of these enzymes. Furthermore, different glycosyltransferases utilize the same sugar nucleotide and similar acceptors to generate different products. As discussed below, these properties of glycosyltransferases present unique problems in determining their specific activities when more than one enzyme is present in a tissue sample.

Currently, most assays for glycosyltransferases use radioisotopes and involve the measurement of radioactivity incorporated from the sugar nucleotide into the product glycosides. A variety of non-radioisotopic glycosyltransferase assays have been developed, but the sensitivity of these assays is not significantly greater than that obtained using the existing radioisotopic assays. Although the sensitivity of the known assays may be high relative to many other types of enzyme assays, it is unacceptably low for many glycosyltransferases, whose activities may be as low as 1 pmol/hr/mg cell protein.

One assay for glycosyl transferases has been reported by the laboratory of one of the present inventors. An assay that uses a lectin to capture specific enzyme products of the enzyme UDP-galactose:$\beta$-D-galactosyl-$\alpha$1,3-galactosyltransferase is reported in Cummings et al., J. Biol. Chem. (1988) 5:511-519. Radioactively labelled galactose was transferred from the donor molecule UDP-GlcNAc (uridine-5'-diphosphate-N-acetylgalactosamine) to the terminus of a glycoprotein acceptor molecule, and the product was separated from the solution using a lectin-derived affinity column that bound the product of the enzyme reaction but neither of the reactants. However, the assay was severely limited by the time and difficulty required to measure the radioactive label.

There have been a number of attempts over the years to eliminate the use of radioisotopes and replace them with other types of detectable labels in immunoassays. Certain enzymes have been successfully used as labels in a number of immunoassays, even though these are generally less sensitive than isotopic labels. Typical of such assays are those in which an immunoglobulin is labeled with an enzyme, commonly refered to as enzyme immunoassays (EIA). It is also possible in such assays to label the analyte with an enzyme. EIAs are described in more detail in Chapter 3 of "Practical Immunoassay," edited by Wilfrid R. Butt, Volume 14 of Clinical and Biochemical Assays, Marcel Dekker, Inc., 1984.

In addition to enzymes, chemiluminescent compounds and bioluminescent compounds have also been used as non-radioactive labels in the place of isotopes in immunoassays. Such assays are refered to as luminescence immunoassays (LIA) or immunochemiluminometric assay (ICMA). These labels have usually proven equivalent in sensitivity to isotopes but have often been hindered by problems associated with non-specific binding of the protein component of the marker (typically a luciferase). These assays are described in further detail of Chapter 5 of "Practical Immunoassay", op. cit., and by Patel and Cormier, Analytical Applications of Bioluminescence and Chemiluminescence, pages 273-276, Academic Press, Inc., 1984.

However, none of the assays previously available has provided the generality, sensitivity, and lack of background interference that is desirable for assays of enzyme activity. As a result, there remains a need for assays that allow direct measurement of enzyme activity, as opposed to enzyme concentration, in a simple and direct manner that is readily adaptable to a wide variety of enzymes.

The present invention seeks to provide a method for specifically, rapidly, and easily measuring enzyme activity, particularly in samples of biological origin, which ameliorates any of the above or other problems.

This invention also seeks to provide a method of measuring enzyme activity that is applicable to a variety of enzymes without requiring major variations in technique.

This has been achieved by providing a method for determing the presence or level of activity of an enzyme in a sample, comprising the steps of combining in a reaction medium

(1) a sample suspected of containing said enzyme and

(2) a labeled substrate for said enzyme capable of reacting with said enzyme to provide a labeled product, allowing a reaction to take place between said enzyme and said substrate whereby said product is formed in the presence of said enzyme; combining with said reaction medium a receptor capable of binding specifically with either said product or said substrate (but not both) to form a complex; and determining the presence or amount of label in said complex or uncomplexed, in said reaction medium wherein said label comprises a bioluminescent protein. The label may comprise a substance which is capable of being converted into a bioluminescent protein label.

The reaction may be allowed to take place for a fixed length of time.

The method may comprise determining the presence or amount of label in said complex or in the uncomplexed, labeled component that does not react with the receptor by triggering emission of light under control of said bioluminescent protein.

The receptor may be a lectin. The receptor may be an antibody. The receptor may be a hormone receptor and said product is a hormone capable of binding specifically with said hormone receptor.

The substrate and said product may comprise carbohydrates. The enzyme may be glycosyltransferase. The receptor may be a lectin.

The label may comprise a luciferase. The label may comprise a photoprotein. The label may comprise aequorin, obelin, mnemiopsin, or berovin. The label may comprise recombinant aequorin. The method may further comprise the step of adding calcium ions to said reaction medium immediately prior to said determining and said determining comprises measuring light emitted by aequorin.

In the method the label may be attached to said substrate by a linker comprising a biotin-avidin or biotin-streptavidin complex. The linker may comprise a substrate portion comprising biotin linked covalently to said substrate. The linker may comprise a label portion comprising biotin linked covalently to said label and said linker further comprises streptavidin noncovalently linking said substrate and label portions.

In order that the invention is more clearly understood specific embodiments will now be described by way of example only (and not by way of limitation) with reference to the drawings that form part of the present specification, wherein:

Figure 1 is a schematic diagram of a first embodiment of the invention;

Figure 2 is a schematic diagram of a second embodiment of the invention;

Figure 3, is a schematic diagram of a third embodiment of the invention;

Figure 4 is a schematic diagram of a fourth embodiment of the invention; and

Figure 5 is a schematic diagram of an embodiment of the invention as applied to a particular assay.

The present invention provides a method for detecting the level of enzyme activity for any enzyme that produces a product that can be distinguished from the enzyme substrate by binding with a binding partner (receptor). By using a receptor that will capture the product without binding to the substrate to an appreciable degree (or vice versa), the amount of product formed or substrate reacted can be readily separated and quantified. The method of the invention also requires a bioluminescent protein, preferably a photoprotein capable of binding a luciferin molecule and being triggered by an external trigger, as the detectable label for the substrate and/or the product. Alternatively, the label may be capable of being convented to a bioluminescent protein label, for example by capturing an avidin complex of the protein label with a biotin molecule attached to the substrate or product. By using bioluminescent proteins as labels various advantages (set forth in detail in later sections of this specification) are obtained.

The method was initially designed using lectins as receptors and carbohydrates as substrates and products for assaying glycosyltransferases, as these enzymes have very low activities because they occur naturally in such low amounts and therefore represent a difficult enzyme-measurement problem. Since the assay was demonstrated to be useful for this difficult assay, it can now easily be applied to other systems. The availability of other natural specific binding compounds, such as hormone receptors, makes the present invention widely applicable to a variety of enzymes. Additionally, the availability of monoclonal antibodies of high specificity through the production of hybridomas allows the techniques to be applied to many different enzyme products for which no natural receptor is available.

The scope of the method is quite broad, as shown below. A sample suspected of containing an enzyme is collected according to standard techniques and combined with a detectably labeled substrate for the enzyme or with a substrate that is capable of being labeled at a later step in the assay. The substrate is also capable of reacting with the enzyme to provide a detectably labeled product (or product capable of being later labeled). The reaction medium in which these principal components are combined contains any additional components that are necessary for the enzyme-catalyzed reaction. For example, if the reaction that the enzyme catalyzes is the covalent joining of another component to the substrate, that other component is provided in the reaction medium. Sufficient time is allowed for a detectable amount of product to be produced, should enzyme be present in the sample being tested. A receptor binding specifically with the product while having substantially no specific binding affinity for the substrate (or vice versa) is combined with reaction medium, either before or after the enzyme-catalyzed reaction is allowed to take place. Depending on the type of reaction being carried out, the product-receptor or substrate-receptor complex is then separated from the reaction medium (heterogenous assays) or a measurement is made on the reaction medium containing the product-receptor or substrate-receptor complex in soluble form (homogenous assays). The presence (for qualitative assays) or amount (for quantitative assays) of detectable label in either the complexed component or the uncomplexed, soluble component is then determined.

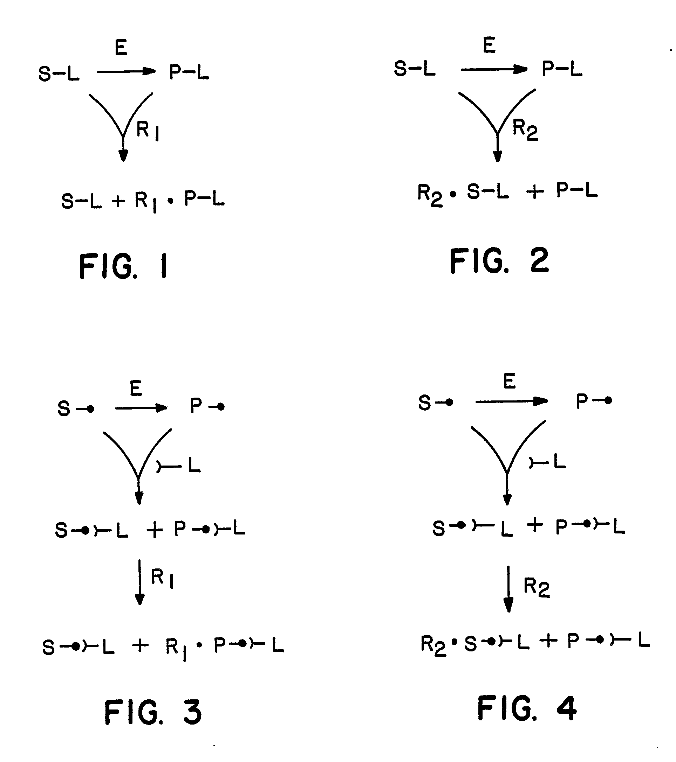

The individual steps of the assay can vary widely in accordance with the different techniques that are available for carrying out heterogeneous and homogeneous assays. Additional variation is possible since the substrate can be labeled with an intermediate label that is converted to a label comprising the bioluminescent protein either before or after the substrate is reacted with the enzyme. Four such variations are shown in Figures 1-4. In Figure 1, a substrate (S) labeled with a bioluminescent protein (L) reacts with an enzyme (E) to produce a labeled product (P) that is then bound by a first receptor ($R_1$). The receptor/labeled-product complex is then separated from the uncomplexed labeled substrate in a heterogeneous assay or a homogeneous assay can be carried out without separation. As shown in Figure 2, a different receptor ($R_2$) is used that complexes with the substrate rather than the product. Homogeneous and heterogeneous assays are likewise possible for this variation. In Figures 3 and 4, substrate labeled with biotin (S—•) is converted to product (P—•) by the enzyme. A bioluminescent protein attached to avidin ( ⊃—L) then added to form the final labeled material. Then either the first receptor is used to form a complex with the product (Figure 3) or the second receptor is used to form a complex with the substrate (Figure 4). Each of these steps, as well as other variations, is described in detail below.

The present method can be used to determine the level of activity of any enzyme that produces a product sufficiently different from its substrate so that a binding interaction with a receptor can take place that distinguishes between the substrate and the product. Because of the high degree of specificity that biological receptors are capable of, practically any enzyme will produce a product that can be distinguished from the original substrate by binding to a receptor. Table 1 below identifies enzymes according to the international classification system. This classification system is referred to elsewhere in this specification.

Table 1

International Classification of Enzymes (Class Names, Code Numbers, and Types of Reactions Catalyzed)

1. Oxido-reductases
(Oxidation-reduction reactions)
    1.1 Acting on -CH-OH

1.2 Acting on -C = O
1.3 Acting on -CH = CH-
1.4 Acting on -CH-NH$_2$
1.5 Acting on -CH-NH-
1.6 Acting on NADH; NADPH
2. Transferases
(Transfer of functional groups)
2.1 One-carbon groups
2.2 Aldehydic or ketonic groups
2.3 Acyl groups
2.4 Glycosyl groups
2.7 Phospate groups
2.8 S-containing groups
3. Hydrolases
(Hydrolysis reactions)
3.1. Esters
3.2 Glycosidic bonds
3.4 Peptide bonds
3.5 Other C-N bonds
3.6 Acid anhydrides
4. Lyases
(Addition to double bonds)
4.1 -C = C-
4.2 -C = O
4.3 -C = N-
5. Isomerases
(Isomerization reactions)
5.1 Racemases
6. Ligases
(Formation of bonds with ATP cleavage)
6.1 C-O
6.2 C-S
6.3 C-N
6.4 C-C

The method of the present invention can be used with enzymes from any of the listed classes or any other enzymes not so listed. As will be apparent from further discussions in the specification, the substrate of the enzyme, the enzyme, the product of the enzyme, and the receptor are all variables that can be modified by one skilled in the art of biochemical analysis, when using the description in this specification, to obtain an acceptable combination of components that can be used in the assay.

The present invention is most easily practiced when a natural receptor exists for either the substrate or product of the enzyme being analyzed. For example, there are a number of specific lectins that recognize terminal portions of carbohydrate chains with a high degree of specificity. By selecting a substrate that is, for example, missing only the terminal carbohydrate in a particular sequence recognized by a lectin, that lectin can be used to bind the product produced when a glycosyltransferase adds the appropriate terminal carbohydrate to the substrate carbohydrate chain. Other examples of natural receptor-enzyme pairs include hormone receptors and enzymes involved in the production of the hormone, and transport proteins (e.g., thyroxine binding globulin) and enzymes that synthesize the transported material.

It is not necessary for the product component of the mixture to be a product produced directly by the enzyme being analyzed. For example, biosynthesis of cholesterol from the lipid squalene is controlled by a series of enzymes. Squalene 2,3-epoxide is cyclized by the enzyme squaleneoxidecyclase to lanosterol. Several more steps are required to convert lanosterol into cholesterol. However, a reaction medium that allows measurement of squaleneoxidecyclase by determining the amount of cholesterol produced can be readily prepared. Such a reaction medium would contain labeled squalene (or any other labeled precursor of squalene 2,3-epoxide, the actual substrate of the enzyme), excess amounts of the enzymes normally required to convert lanosterol to cholesterol so that the step controlled by squaleneoxidecyclase is the limiting step of the reaction, and any cofactors necessary for production of cholesterol or any of the intermediates. Other enzyme systems can be manipulated in a similar manner to provide an acceptable product, substrate, and receptor combination to make accessable the measurement of the activity of any enzyme in a metabolic or other type of enzyme-controlled pathway. Accordingly, the terms "substrate" and "product" as used herein do not necessarily refer to the immediate substrate of the enzyme being analyzed or to its immediate product but can refer to another substrate or product in a pathway involving the enzyme being analyzed.

If a natural receptor is not available for the substrate or product, an appropriate receptor can be prepared by techniques of immunology. Monoclonal antibodies are preferred because of their high specificity, although antisera can be used in appropriate situation. In preparing both monoclonal antibodies and specific antisera, B cells (either in vivo or in vitro) are stimulated with the reaction component to which binding is desired (either the substrate or the product). Unlabeled components are typically used to avoid stimulation of antibody production against the label, which is usually present in both the product and the substrate. An antiserum, or a cell that produces an antibody in the monoclonal process, is then selected by detecting the ability of antibodies to bind with the component with which binding is desired. A second screening reaction can also be used to eliminate binding reactions between the antibodies and the non-selected component (e.g., the substrate if the receptor is intended to bind with the product). For example, the substrate can be attached to an affinity column, where it will remove antibodies from a solution passed through the column if these antibodies have binding affinity for the substrate. If a labeled component has been used as the immunogen, this second selection process can also be used to eliminate antibodies that bind with the label portion of a molecule. Production of monoclonal bodies by preparation of hybridomas is a well developed procedure and need not be described in detail at this time.

The reaction medium in which the reaction between the enzyme and substrate takes place is typically an aqueous solution buffered at a pH appropriate for the enzyme being analyzed. The reaction medium also contains other components necessary for the reaction to take place. For example, if the enzyme is a transferase, the reaction medium would also contain the component to which the substrate is being transferred. If the enzyme is an oxido-reductase, a source or acceptor of hydrogen ions (e.g., NADH) would also be provided. Other components of the reaction medium will be apparent from the nature of the enzyme reaction being analyzed.

The sample can be any sample suspected of containing the enzyme being analyzed and will be handled in accordance with standard procedures relating to the type of sample being collected. Typical samples include blood (either as serum, plasma, or whole blood), urine, saliva, feces, tissue samples, and the like. The sample can be used either in the form in which it is originally obtained or it can be subjected to various processing steps to provide the enzyme in a form suitable for assay. For example, if the enzyme is normally found in the cytoplasm of cells, the cell walls can be disrupted to release the enzyme into the reaction medium.

The substrate on which the enzyme acts is selected with two principal features in mind. The first of these is the ability of the enzyme to produce a product from the substrate that is distinguishable from the substrate by a specific binding reaction with the receptor. Because of the high specificity of biological binding systems, this will not normally be a problem. Whether any particular substrate is suitable for an assay can readily be determined by measuring the binding affinity of the selected receptor for both the substrate and the enzyme reaction product. Radioactive labels, e.g., non-specific tritium labels, are satisfactory for this type of screening, should it be necessary,

The second principal requirement of the substrate is its ability to be detectably labeled by a bioluminescent protein in a manner tha does not interfere with binding between the substrate and the enzyme. Labelling of biological substances by numerous types of detectable markers is a well developed area of clinical chemistry. Bioluminescent markers are well known, as are techniques by which such labels can be attached to other molecules. Numerous publications and patents describe techniques for labeling many different kinds of compounds. Examples that describe attachment of bioluminescent labels to analytes include U.S. Application Serial No. 059,137, filed June 5, 1987 (equivalent to European publication number EP-A-137515) entitled "Bioluminescent Immunoassays Utilizing Coelenterate-Derived Luciferases and Photoproteins," U.S. Patent No. 4,478,817 entitled "Detecting or Quantifying Substances Using Labelling Techniques," and U.S. Patent No. 4,604,364, entitled "Bioluminescent Tracer Composition and Method of Use in Immunoassays."

The particular bioluminescent label used in the method of the present invention comprises a protein or related molecule (e.g., glycoprotein) capable of binding to a luciferin or other light emitting molecule and inducing light emission under appropriate conditions. Surprisingly, it has been found that bioluminescent proteins can be attached to enzyme substrates without adversely affecting the ability of the substrate to react with the enzyme or the ability of the bioluminescent protein to emit light efficiently. Additionally, bioluminescent proteins, when derivatized and used as a label, are found to have higher quantum yields than chemiluminescent labels. Although bioluminescent proteins may appear to be equivalent to other labels, such as radioactive labels and chemiluminescent labels, in practice many compounds of biological interest cannot be detected without the use of bioluminescent proteins as labels.

A variety of bioluminescent protein labels are available. U.S. Patent No. 4,478,817, U.S. Patent No. 4,604,364, and U.S. Patent Application Serial No. 059,137, filed June 5, 1987 all of which are cited above in more detail, describe bioluminescent protein labels that can be used in the practice of the invention. Additionally, Serio and Pazzagli, eds. Luminescent Assays Volume 1, Raven Press, 1982, pages 115 and 125, give examples in which firefly luciferase is used as a label in heterogenous immunoassays for the detection of picomole quantities of methotrexate and trinitrotoluene.

It is preferred to use recombinantly prepared proteins as the bioluminescent labels of the present invention. U.S. Patent Application Serial Nos. 165,422, filed February 29, 1988, and 173,045, filed March 17, 1988 (both equivalent to European publication number EP-A-187519) describe a number of recombinant DNA vectors capable of expressing apoaequorin, a coelenterate-derived photoprotein.

Particularly preferred as protein labels are photoproteins (as opposed to luciferases and other bioluminescent protein that act in an enzymatic manner). Photoproteins of the invention are readily distinguishable from enzymes such as luciferase that control luminescent reactions, as the photoproteins have the ability to bind the actual luminescent molecule (e.g., a luciferin) in the presence of oxygen without immediately emitting light.

Coelenterate-derived photoproteins are particularly useful for the practice of the present invention. Examples of coelenterate-derived photoproteins are aequorin, obelin, mnemiopsin, and berovin. These molecules are described in a number of technical publications, including Ward et al., Proc. Natl. Acad. Sci. USA (1975) 72:2530-2530. Such coelenterate-derived proteins are stable in the absence of free calcium ions and contain both coelenterate luciferin and oxygen bound to the protein. The term "luminescent molecule" is used herein to refer to luciferin and similar small organic molecules bound to photoproteins (or acted upon by luciferase), which are the actual sources of the emitted light. When calcium ions, a bioluminescence trigger for coelenterate-derived photoproteins, are added to solutions labeled with the photoprotein, a blue light is emitted by the luciferin molecule, having a emission maximum at a wave length of about 469 nm. Such labels are described in detail in U.S. Application Serial No. 059,137, filed June 5, 1987, which is herein incorporated by reference. A form of the photoprotein prepared by techniques of genetic engineering is described in U.S. Applications Serial No. 165,422, filed February 29, 1988, and 173,045, filed March 17, 1988, both of which are entitled "Recombinant DNA Vectors Capable of Expressing Apoaequorin."

A particular advantage that is available for photoproteins as described herein is their ability to be used without a bound luminenscent molecule in a technique for reducing background luminescence. One of the problems associated with using luciferase and other enzymatic molecules that react immediately with luminescent molecules to emit light is the propensity of the proteinaceous components of such systems to bind nonspecifically to various surfaces and molecules present in the reaction medium. When luciferin or another luminecent molecule is then added to the medium in order to produce light, background luminescence occurs. Since photoproteins require a separate trigger molecule, photoproteins not containing the luminescent molecule can be used to precoat surfaces in the reaction medium. Such proteins are referred to as apophotoproteins. A photoprotein charged with the luminescent molecule is then used as the label. Since the conditions under which loading and unloading of the luminescent molecule onto and off of the apophotoprotein occur can be controlled, it is possible to select conditions for the reaction medium so that no exchange of the luminescent molecule between photoproteins occurs. Under these circumstances, only photoproteins used as labels will emit light when triggered by their external trigger (calcium ions for coelenterate-derived photoproteins) since the apophotoproteins used to eliminate background luminescence do not contain a luminescent molecule. This advantageous technique that can be used with preferred embodiments of the invention (and a number of variations thereof) are described in a co-pending application filed on the same day as the present application and entitled "Method For Increasing Sensitivity in Luminescence Assays."

After the reaction medium containing the enzyme and detectably labeled substrate has been prepared, sufficient time is allowed for a reaction to take place between the enzyme and the substrate. Since enzymes have different turnover rates, this time will vary with the individual enzyme being analyzed. Typical rates can vary from a few seconds to a few minutes with even longer incubation periods being allowed for slow-acting enzymes.

Since enzyme activity is generally measured by determining the amount of reaction that takes place in a given amount of time, some method of accurately beginning and ending the time measurement is necessary. In most cases the reaction will be initiated by the mixing of the sample with the labeled or unlabeled substrate. The reaction can be ended in a number of ways, depending on the particular enzyme and/or type of assay being used. For example, the pH of the reaction medium can be changed (for example, by adding acid) to provide a pH at which enzyme activity is greatly reduced. Other techniques for stopping the reaction include addition of a large excess of inhibitor, heating the reaction medium to denature the enzyme, cooling the medium to reduce the reaction rate, and adding a surfactant or other caotropic agent to disturb the enzyme structure. If a reaction is carried out in which the product is captured by a solid-phase receptor attached to a solid-phase surface (referred to as a solid-phase receptor), the reaction can be stopped by removing the reaction medium from contact with the solid-phase receptor. For example, if the receptor is attached to the walls of a microtiter plate well, the reaction medium can be quickly aspirated and washed either manually or in an automated microtiter plate processor. If a receptor is being used that binds with the enzyme substrate rather that the product, adding receptor to the reaction medium will cause the substrate to bind with the receptor, thereby preventing it from reacting with the enzyme. In this and similar cases where the enzyme is not deactivated, however, care must be taken to prevent the equilbrium from being disturbed, since the enzyme is occasionally capable of catalyzing the reverse reaction (in this case, formation of substrate from product) so that if continued contact is allowed between the enzyme and the non-captured component of the reaction, anamolous results may be obtained. This is not a problem in many reactions since other reactions may be taking place in the reaction medium to remove the potentially reactive component, e.g., hydrolosis of products or their conversion into a form not capable of undergoing the reverse reaction. Other variations for stopping the reaction will be apparant to those skilled in the art.

At an appropriate time, which will vary depending on the assay being used, a receptor capable of binding specifically with either the detectably labeled product to form a product-receptor complex or the detectably labeled substrate to form a substrate-receptor complex is combined with the reaction medium. As indicated previously, the receptor is chosen so that there is substantially no specific binding affinity for one of the two components and specific binding affinity for the other. Absolute values of binding affinity are relatively unimportant. However, higher binding affinities increase the sensitivity of the assay and allow smaller quantities and activities of the enzyme to be measured. Specific binding affinity, as opposed to non-specific binding affinity, refers to the ability of a receptor to interact at its natural binding target molecule at its natural binding site. Association constants ($K_A$; expressed in units of $M^{-1}$) for the interaction of a receptor (R) and target molecule (T) to form a complex (R-T; also expressed here as C) in the reaction $R + T \rightarrow C$ are generally at least $10^7$ for antibody-target (ligand) interactions. Association constants for such antibody binding reactions are known as high as $10^{12}$. Association constants for other types of ligand-receptor interactions can be significantly higher. For example, the biotin-avidin interaction has an association constant of approximately $10^{15}$. Hormone-receptor interactions often approached or even exceed this last value.

More important than the absolute value of the association constant is the ratio of the association constant of the desired target to the association constant of the other component to which binding is not desired. For example, if binding is desired to the product, it is prefered that the association constant for this binding reaction be at least $10^3$ times the association constant for the interaction between the receptor and the substrate. More preferably, this ratio is at least $10^5$, even more preferably at least $10^7$. Relative binding affinities can readily be determined by competition between product and substrate for a limited number of receptor binding sites (in the absence of enzyme).

If desired, the bioluminescent protein can be attached to the product or substrate after product formation by the enzyme. While this attachment can be carried out if desired in the same manner in which the label is attached to a substrate prior to enzyme reaction, it is preferred to use a noncovalent linking group to allow attachment under the conditions that would be present in the reaction medium in which the enzyme reaction takes place. For example, the strong binding reaction between biotin and avidin can be used to link the label to a product in solution after formation. Avidin, preferably streptavidin, can be bound to the substrate either directly or indirectly, as when biotin is directly bound to the substrate and avidin binds to the biotin on the substrate. A biotinylated luminescent agent, for example, biotinylated aequorin, can then be added to the solution so that the substrate becomes labeled with the photoprotein. Several techniques for using avidin-biotin complexes to attach labels to organic molecules are described in Hsu et al., in "Immunoperioxidase Techniques: A Comparison Between ABC & Unlabeled (PAP) Procedures" (1981) J. Histochem. Cytochem. 29: 577.

Once a binding interaction has taken place between the receptor and the appropriate component of the reaction medium, steps are taken to detect the presence or amount of detectable label in one of the two detectable forms to be measured. Because of the wide variety of measurement types to which the present assay may be applied, these techniques can vary widely. For example, if the assay is a homogeneous assay, quenching or energy transfer can be used to distinguish label that is complexed with a quencher- tagged (or energy-receiver-tagged) receptor from label that is bound to only a soluble substrate or product molecule. If the reaction is a heterogeneous assay, in which the solid and liquid phases are to be separated, and the target to which the receptor binds is the labeled product, then separation of the reaction medium from the solid-phase receptor complex allow measurement of the label in either the solid-phase receptor complex or the liquid-phase, uncomplexed, detectably labeled substrate. The specific steps that will be necessary for determining the presence or amount of the detectable label and one of the two components will be readily apparent to one skilled in clinical chemistry from the type of label being used and the specific type of assay (e.g., homogeneous or heterogeneous, measurement of product or unreacted substrate). When a photoprotein is used as the label, luminescence is measured after adding an appropriate trigger to the medium being measured in order to induce luminescense to occur.

As an example of the types of assays that are possible, measurement of glycosyltransferases by the method of the invention is discussed here in more detail. In addition to providing further detail as to how the assay would be carried out, this material also shows the use of the enzyme capture assay of the invention to measure enzyme activity which was not readily accessible by previous measurement techniques.

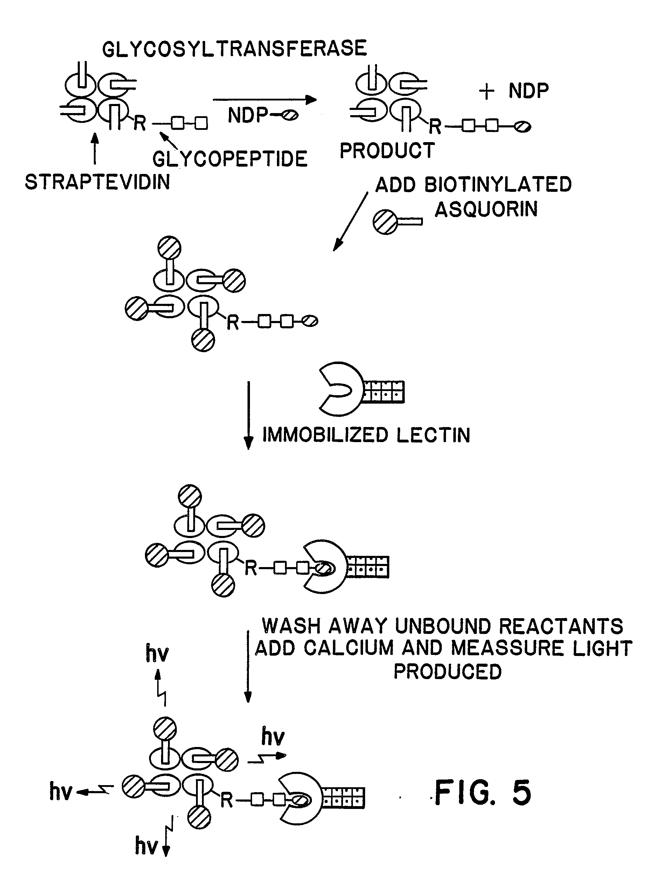

In this example, the enzyme being analyzed is UDPGal:$\beta$-D-GlcNAc($\beta$1,4)galactosyltransferase. The principal components involved in this assay are shown in Figure 5. As illustrated in the Figure, two primary reagents used in the assay are recombinant aequorin and streptavidin. Native aequorin is a 20,000 dalton bioluminescent protein from the jellyfish Aequorea victoria. Each mole of the protein contains approximately one mole of the coelenterate chromophore luciferin. The protein produces blue light ($\lambda_{max}$ 469 nm) in the presence of calcium ions, and the quantum yield is such that aequorin is detectable in a range of $10^{-19}$ to $10^{-20}$ mole. This amount of aequorin is equivalent to approximately 1,000 - 10,000 molecules per liter. The gene coding apoaequorin (the protein in the absence of the luciferin molecule) has been cloned, and the protein is available as "recombinant" apoaequorin, as described above. Recombinant apoaequorin is converted to recombinant aequorin in high yield upon incubation with synthetic coelentrate luciferin, dissolved oxygen, and 2-mercaptoenthanol. The recombinant aequorin is biotinylated using commercially available reactants. The second reagent, streptavidin, is a 60,000 dalton biotin-binding protein from Sterptomyces avidinii. Each molecule of this protein binds four molecules of biotin with high avidity ($K_D$ of approximately $10^{-15}$ M). Streptavidin is commercially available.

In the exemplary assay shown in Figure 5, a glycopeptide containing a terminal GlcNAc residue is covalently attached to streptavidin and used as the acceptor for the enzyme UDPGal:$\beta$-D-GlcNAc($\beta$1,4)-galactosyltransferase. The product of the enzyme reaction contains a Gal$\beta$1-4GlcNAc linkage. After incubation, biotinylated recombinant aequorin is added to the reaction mixture where it complexes with all available biotin binding sites on the streptavidin in both the product and the substrate. To isolate the product of this enzyme reaction from the substrate and other components in assay, the reaction mixture is passed over a column of immobilized lectin Ricinus communis agglutinin-I. This plant lectin binds with high affinity to glycoconjugates containing the terminal sequence Gal$\beta$1-4GlcNAc-glycopeptide but does not bind to isomers of this structure, such as Gal$\beta$1,3GlcNAc-glycopeptide or Gal$\alpha$1, 4GlcNAc-glycopeptide. The glycopeptide-streptavidin product bound by the lectin is released from the lectin column by eluting the column with buffer containing lactose, which is a competitive ligand for the lectin. The total amount of product is then determined by measuring the number of photons produced by aequorin upon addition of calcium ions to the eluted solution. Photon emission in this system occurs within ten seconds, and the total photons produced are directly related to the activity of enzyme present in the original sample.

The glycopeptide acceptor (substrate) for the glycosyltransferase can be prepared from fibrinogen. Briefly, fibrinogen glycopeptides, prepared by pronase digestion of fibrinogen, is used as the starting material. The biantennary chains present in fibrinogen glycopeptides are isolated by affinity chromatography on a Con A column. Unbound material is washed off and bound glycopeptide eluted with an $\alpha$-methylmannoside solution. After a series of manipulations, described in detail in the experimental section that follows, an asialo, agalactofibrinogen glycopeptide is obtained. This material, which acts as the substrate in the reaction, is covalently coupled to streptavidin using commercially available reagents as described in detail in the examples that follow.

For this enzyme assay, the reaction mixture contains the streptavidin-glycopeptide conjugate (SGp), UDP-Gal, mangenese chloride, and a buffer. Reactions are carried out at 37°, after which reaction is ended by cooling the mixture to 4° C to stop enzyme activity prior to addition of biotinated aequorin as indicated above. As shown in the following examples, results of this assay indicate that the technology can be used to increase the sensitivity of the determination of galactosyltransferase activity by at least three orders of magnitude relative to classical radioisotope methods previously available.

The same technology can be used for other glycosyltransferases, such as UDPGal:$\beta$1,4Glc(NAc)-($\beta$1,3)-galactosyltransferase and CMPNeuAc:Gal$\beta$1,3(4)Glc-NAc($\alpha$1,3)sialyltransferase. The enzyme UDPGal:βl,4Glc-(NAc)(β1,3)galactosyltransferase attaches galactose in an α1,3 linkage to terminal galactose residues in oligosaccharides containing the sequence Galβ1,4Glc(NAc)-saccharide. The enzyme is responsible for the synthesis of terminal Galα1-3Gal sequences on glycoconjugates. All human sera contain large quantities (about 1% of the circulating IgG) of naturally occuring antibody against this epitope. It has been suggested that anomolous expression of this glycosyltransferase in humans would result in synthesis of the antigenic epitope and possible initiation of autoimmune processes. The basic assay for this galactosyltransferase would be the same as that for the UDPGal:β-D-GlcNAc(β1,4)galactosyltransferase described above. Variations include the structure of the acceptor, the specificity of the immobilized lectin affinity column for the separation of acceptor and product, and the sugar nucleotide. The sugar nucleotide (UDPGal) is the same, but the acceptor glycopeptide will be the asialo derivative of the fibrinogen glycopeptide coupled to streptavidin, and the lectin column will be prepared from the lectin obtained from Griffonia simplicifolia I. This lectin binds to oligosaccharides having the terminal sequence Galα1,3 Gal-saccharide. The bound glycopeptide-streptavidin product will be eluted with methyl-α-galactoside or an oligosaccharide terminating in an α-linked galactose, such as raffinose.

The same assay (with minor variations) can also be used for the enzyme CMPNeuAc:Galβ1,3(4)-GlcNAc-(α1,3)sialyltransferase which transfers NeuAc from CMPNeuUAC to oligosaccharides containing the terminal sequence Galβ1,3(4)GlcNAc-saccharide. The enzyme CMPNeuAc:Galβ1,3(4)GlcNAc(α1,3)-sialyltransferase is an important enzyme in the chain termination reactions during biosynthesis of animal cell glycoconjugates. The product of this reaction, NeuAcα2-3Gal-saccharide, is the cell-surface receptor for influenza virus (H3 serotype) isolated from human cells. Variations in the assay from that described above for UDPGal:β-D-GlcNAc-(β1,4)galactosyltransferase include use of CMPNeuUAC as the sugar nucleotide and the asialo derivative of the fibrinogen glycopeptide as the acceptor molecule. The lectin column will be prepared with the leukoagglutinating lectin from Maackia amurensis, which binds oligosaccharides having the terminal sequence NeuAcα2,3Galβl,3GlcNAc-saccharide, which is the product of this enzyme. Binding of the asialo glycopeptide-streptavidin-aequorin product by this immobilized lectin will be used to separate it from the original substrate and other products, such as those made by related but different enzymes.

Although the examples described above all involve glycosyltransferase enzymes, the considerations required for applying this techniques to any enzyme will be readily apparent to one skilled in the art from the description above and the following examples. There is no theoretical reason for limiting the reaction to exclude any of the classes set forth in Table 1 above or indeed to any known enzyme, as long as the enzyme product can be distinguished from the enzyme substrate by a receptor, as described herein.

The following examples are offered by way of illustration and not by way of limitation.

## EXAMPLE

Assay for UDPGal:β-D-GlcNAc(β1,4)galactosyltransferase activity

Studies to verify the feasibility of the assay were performed with the commercially available (Sigma Chemical Co., St. Louis) enzyme UDPGal:β-D-GlcNAc(β1,4)galactosyltransferase. The principle of the assay is shown in Figure 5.

In the assay a glycopeptide containing a terminal GlcNAc residue was covalently attached to streptavidin and used as the acceptor for the enzyme UDPGal:β-D-GlcNAc(β1,4)galactosyltransferase. The product of the enzyme reaction contains a Galβ1-4GlcNAc linkage at its terminus. After incubation, biotinylated recombinant aequorin was added to complex with all available biotin binding sites on the streptavidin in the product and in the reactants. To isolate the product of this enzyme reaction from other reactants in the assay, the reaction mixture was passed over a column of immobilized lectin Ricinus communis agglutinin-I. This plant lectin binds with high affinity to glycoconjugates containing the terminal sequence Galβ1,4GlcNAc-glycoconjugate but does not bind to isomers of this structure, such as Galβ1,3GlcNAc-glycoconjugate or Galα1,4GlcNAc-glycoconjugate (Merkle and Cummings, Methods in Enzymol. (1987) 138:232-259). The glycopeptide-streptavidin product bound by the lectin was released from the lectin column by eluting the column with buffer containing 100 nM lactose, which is a competitive ligand for the lectin. The total amount of product was then determined by measuring the amount of photons produced by aequorin upon the addition of Ca$^{++}$. Photon emission occured within 10 seconds, and the total photons produced was directly related to the amount of enzyme present in the sample.

Glycopeptide acceptor for galactosyltransferase was prepared from fibrinogen as described previously (Cummings and Kornfeld, J. Biol. Chem. (1982) 257:11235-11240). Briefly, fibrinogen glycopeptides, prepared by pronase digestion of 1 gram of fibrinogen (Sigma Chem. Co.), were used as the starting material. The biantennary chains present in fibrinogen glycopeptides were isolated by chromatography on a 100 ml column of Con A-Sepharose. Unbound material was washed with TBS (10 mM Tris, 1 mM CaCl₂, 1 mM MnCl₂ and 0.02% NaN₃, pH 8.0). Bound glycopeptide eluted with 10 mM α-methylmannoside in TBS was pooled and desalted by chromatography on a column of Sephadex G-25 to remove salts and haptenic sugars. The glycopeptide in the void volume, detected by the phenol-sulfuric acid assay, was pooled and dried. It carries the typical biantennary N-linked oligosaccharides containing terminal sialic acid and lacking fucose residues as shown below.

$$NeuAc\alpha 2-3Gal\beta 1-4GlcNAc\beta 1-2Man\alpha 1-6$$

$$Man\beta 1-4GlcNAc\beta 1-4GlcNAc\beta Asn$$

$$NeuAc\alpha 2-3Gal\beta 1-4GlcNAc\beta 1-2Man\alpha 1-3$$

Sialic acid was removed from the glycopeptide by mild acid hydrolysis and the resulting asialo derivative was digested exhaustively with jackbean β-galactosidase to obtain the desialylated, degalactosylated fibrinogen glycopeptide shown below.

$$GlcNAc\beta 1-2Man\alpha 1-6$$

$$Man\beta 1-4GlcNAc\beta 1-4GlcNAc\beta Asn$$

$$GlcNAc\beta 1-2Man\alpha 1-3$$

The asialo, agalacto fibrinogen glycopeptide was covalently coupled to streptavidin. Briefly, the glycopeptides were dried in an eppendorf tube and allowed to react with 40 μl of 50 μmol/ml ninhydrin solution in sodium acetate buffer, pH 6.0. The product was applied to a column of Bio Gel P10 (1 x 20 ml) to separate activated glycopeptides from Rheumann's purple and unreacted ninhydrin. The sample, lyophilyzed from the void volume of the column, was removed as soon as it was dry and resuspended in 20 μl of 0.17 M KH₂PO₄, pH 7.5. Streptavidin (5 mg) was dissolved in 25 μl of 0.17 M KH₂PO₄, pH 7.5, 5 μl of a solution of NaCNBH₃ in KH₂PO₄ (0.082 g NaCNBH₃/ml 0.17 M KH₂PO₄, pH 7.5) and added to the activated glycopeptides. The mixture was incubated overnight at room temperature, and the streptavidin-glycopeptide conjugate (SGp) was affinity purified on the ConA column and stored frozen in a solution of 100 mM cacodylate buffer, pH 6.5.

For the enzyme assays, the reaction mixtures (35 μl) contained 2 μg SGP, 50 nmol UDP-Gal and 1 μmol MnCl₂, in cacodylate buffer, pH 6.5. Enzyme units used in assays ranged from 1-10 μU. The reaction mixtures were incubated at 37° C for a maximum of 2 h, and the reactions were terminated by adding 20 μl of 200 mM EDTA, pH 8.0. At the end of the incubation, the reaction mixtures were cooled to 4° C, and 1 μg of biotinylated-aequorin (BAEQ) was added. This mixture was incubated on ice for 30 min. The samples were applied to columns of immobilized Ricinus communis agglutinin I, and bound galactosylated SGP-BAEQ product was collected from the column by lactose elution. Portions (500 μl) of the bound fractions were placed in vials, and the product was detected as photons released in a Berthold photometer by injecting 100 μl of calcium-containing assay buffer (0.1 M Tris, 0.1 M CaCl₂, 0.004% NaN₃, pH 7.5).

An experiment has been carried out to determine total photons produced as a function or enzyme activity. Photons were converted to femtomols of biotinylated aequorin-associated product produced. This conversion was accomplished using absolute light standards constructed from measurements made using known concentrations of photoproteins. These results indicate that this assay technology can be used to increase the sensitivity of the determination of glycosyltransferase activities by at least three orders of magnitude relative to classical radioisotopic methods without the necessity of expensive radiolabeled reagents and the associated radioactive waste problems,

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. A method for determining the level of activity of an enzyme in a sample, comprising:
a. combining in a reaction medium
(1) a sample supected of containing said enzyme and
(2) a labeled substrate for said enzyme capable of reacting with said enzyme to provide a labeled product;
b. allowing a reaction to take place in said reaction medium between said enzyme and said substrate, whereby said product is formed in the presence of said enzyme;
c. either before or after said reaction is allowed to take place, combining said reaction medium with a receptor capable of binding specifically with either said product or said substrate, but not both, to form a complex;
d. determining the presence or amount of a label in said complex or uncomplexed in said reaction medium, wherein said label comprises a bioluminescent protein.

2. The method of Claim 1, wherein said receptor is attached to a solid surface, whereby a solid-phase complex forms between said receptor and said detectably labeled product or substrate.

3. The method of Claim 2, wherein said labeled substrate is present in a liquid-phase reaction medium and said solid-phase complex is separated from said liquid-phase reaction medium prior to said determining.

aequorin.

4. The method of Claim 1, wherein said said receptor is a monoclonal antibody that bind said product with an affinity at least $10^5$ times that with which said antibody binds said substrate.

5. In a method for determining the level of activity of an enzyme in a sample in which a sample suspected of containing said enzyme is combined in a reaction medium with a substrate capable of reacting with said enzyme to form a product and the amount of product formed or substrate reacted is determined as a measure of enzyme activity, the improvement which comprises:
distinguishing product from unreacted substrate by binding of said product or said substrate with a receptor having specific binding affinity for said product or substrate, wherein said binding forms a complex distinguishable from uncomplexed substrate and product; and
detecting said product or said substrate in said complex or uncomplexed in said reaction medium using a bioluminescent protein label on said product or said substrate.

6 The method of Claim 5 , wherein said bioluminescent protein is a photoprotein.

7. The method of Claim 6, wherein said photoprotein in aequorin.

8. The method of any one of claims 5 to 7 wherein said complex comprises product and receptor in the substantial sbsence of said substrate.

9. The method of Claim 8 wherein said substrate is attached to said label with a linking group comprising a biotin-avidin or biotin-streptavidin complex.

10. The method of Claim 9, wherein said receptor is a lectin.

11 The method of Claim 10 wherein said product and said substrate are biotin-tagged carbohydrates and said bioluminescent protein label is attached to said product or said substrate after said reacting by binding said biotin to a complex comprising said bioluminescent protein and avidin.

$$S-L \xrightarrow{E} P-L$$

$$S-L + R_1 \cdot P-L$$

**FIG. 1**

$$S-L \xrightarrow{E} P-L$$

$$R_2 \cdot S-L + P-L$$

**FIG. 2**

$$S-\bullet \xrightarrow{E} P-\bullet$$

$$S-\bullet\rangle-L + P-\bullet\rangle-L$$

$$\downarrow R_1$$

$$S-\bullet\rangle-L + R_1 \cdot P-\bullet\rangle-L$$

**FIG. 3**

$$S-\bullet \xrightarrow{E} P-\bullet$$

$$S-\bullet\rangle-L + P-\bullet\rangle-L$$

$$\downarrow R_2$$

$$R_2 \cdot S-\bullet\rangle-L + P-\bullet\rangle-L$$

**FIG. 4**

GLYCOSYLTRANSFERASE

STRAPTEVIDIN

GLYCOPEPTIDE

PRODUCT + NDP

ADD BIOTINYLATED ASQUORIN

IMMOBILIZED LECTIN

WASH AWAY UNBOUND REACTANTS
ADD CALCIUM AND MEASSURE LIGHT
PRODUCED

FIG. 5